# EUROPEAN PATENT APPLICATION

(11) **EP 4 748 403 A1**
(43) Date of publication of application: **27.05.2026**
(21) Application number: 24842900.3
(22) Date of filing: 25.06.2024
(51) Int. Cl.: A61L 2/20, A61L 2/025

(54) **DECONTAMINATION SYSTEM**

(30) Priority: 19.07.2023 JP 2023117251
(71) Applicant: Airex Co., Ltd., Nagoya-shi Aichi 453-0015 (JP)
(72) Inventor: KAWASAKI, Koji, Nagoya-shi Aichi 453-0015 (JP); OGATA, Yoshitaka, Nagoya-shi Aichi 453-0015 (JP); KAKUDA, Daisuke, Nagoya-shi Aichi 453-0015 (JP); KITANO, Tsukasa, Nagoya-shi Aichi 453-0015 (JP); GUO, Zhiqiang, Nagoya-shi Aichi 453-0015 (JP)
(74) Representative: Kraus & Lederer PartGmbB
(86) International application number: PCT/JP2024/022951
(87) International publication number: WO 2025/018104

(57) **Abstract**

The present invention provides a decontamination system capable of continuously decontaminating numerous accommodating containers in small equipment because the consumption of a hydrogen peroxide solution can be reduced within a required range, leading to a smaller amount of the hydrogen peroxide residual inside each accommodating container and significantly shortened decontamination and aeration durations.

The present invention is characterized by a continuous supplying device used in a decontamination agent supply step.

The continuous supplying device includes an ejection means, a heating means, and an ultrasound emitting means on a conveyance path of a conveyance means.

The hydrogen peroxide solution ejected onto a surface of a sealing member of the accommodating container by the ejection means increases in temperature by the heating means and allows hydrogen peroxide to vaporize with ultrasounds emitted from the ultrasound emitting means and a vaporizing hydrogen peroxide gas is supplied from the surface of the sealing member to the inside of the accommodating container by receiving ultrasonic sound flows.

## Description

### TECHNICAL FIELD

The present invention relates to a decontamination system for decontaminating external surfaces of an article being accommodated inside an accommodating container.

### BACKGROUND ART

The convenience of the clinical environment allows for the production of pre-filled syringes filled with a pharmaceutical product. In this case, each pharmaceutical product is filled in syringes in a filling working chamber in a sterile environment. However, pre-filled syringes are normally accommodated in an accommodating container in a clean environment such as a clean room, but the sterile level inside the accommodating container cannot be strictly guaranteed.

To eliminate such a drawback, pre-filled syringes are accommodated, using a conventional type of accommodating container including a plastic container body molded according to the shape of a pre-filled syringe and a gas-permeable sealing member. The sealing member used is generally Tyvek (trademark) as a non-woven fabric composed of high-density polyethylene microfibers, and the Tyvek (trademark) is gas-permeable through micropores included in the Tyvek (trademark) product to the inside of the plastic container, but blocks ingress of microorganisms. In this case, while pre-filled syringes are being accommodated in an accommodating container, external surfaces of each of the pre-filled syringes and the inside of the accommodating container are decontaminated with a decontamination agent such as hydrogen peroxide gas.

Nevertheless, after hydrogen peroxide gas is supplied to the inside of the accommodating container via the gas-permeable sealing member for decontamination, the accommodating container needs to be left unattended for a significantly longer period of time to completely remove the residual hydrogen peroxide inside the container. Unfortunately, this process deteriorates the efficiency of decontamination operations and lowers the productivity of pre-filled syringes accommodated in the accommodating container. To solve this problem, the following Patent Document 1 proposes a sterilization method capable of assuredly removing the hydrogen peroxide gas residual after decontamination with hydrogen peroxide gas, with pre-filled syringes being accommodated inside an ultrasonic accommodating container.

### CITATION LIST

### PATENT LITERATURE

Patent Document 1: JP-A-11-193010 A

### SUMMARY OF INVENTION

### TECHNICAL PROBLEM

In fact, a method described in the above Patent Document 1 is to repeat an operation of allowing hydrogen peroxide gas to permeate the inside of an accommodating container under negative pressure one or more times. This method further sterilizes the inside of the accommodating container with hydrogen peroxide gas, and thereafter removes the hydrogen peroxide gas from the inside of the accommodating container by heating the container. However, this method inevitably requires a large negative-pressure chamber. Another problem with decontamination in a negative-pressure chamber is that repetitive operations of negative pressure and pressure restoration are needed, thereby taking a longer decontamination duration. The negative-pressure operation can also allow air bubbles to be mixed into chemicals filled in pre-filled syringes. Further, with accommodating containers overlapped for decontamination, the shape of such containers must correspond to uses and may be determined in a limited manner.

Thus, the present invention was made in view of the situation to solve the problems, and has an object to provide a decontamination system capable of continuously decontaminating numerous accommodating containers in small equipment because the consumption of a hydrogen peroxide solution can be reduced within a required range, leading to a smaller amount of the hydrogen peroxide residual inside each accommodating container and significantly shortened decontamination and aeration durations.

### SOLUTION TO PROBLEM

To solve the aforementioned problem, inventors of the present invention have carried out an extended investigation to find that hydrogen peroxide gas can be supplied to the inside of an accommodating container via a sealing member by uniformly applying a small amount of hydrogen peroxide solution over a surface of a gas-permeable sealing member of the accommodating container and irradiating with ultrasound the surface of the sealing member. Based on that technique, the present invention was accomplished.

Specifically, a decontamination system according to the present invention is, according to description in claim 1,
a decontamination system for decontaminating external surfaces of a pre-filled syringe (S) accommodated inside an accommodating container (P), composed of a container body (P1) and a gas-permeable sealing member (P2) for sealing a released portion of the container body, with the pre-filled syringe being accommodated, the system comprising:
a decontamination agent supply step of supplying a decontamination agent to the inside of the accommodating container using a continuous supplying device (100); a decontamination step of maintaining the accommodating container supplied with the decontamination agent for a predetermined duration and decontaminating external surfaces of the pre-filled syringe; and an aeration step of removing the residual decontamination agent from the inside of the accommodating container after decontamination and replacing the agent with clean air, characterized in that
the continuous supplying device includes a conveyance means (10) for conveying the accommodating container from an inlet (111) to an outlet (131), an ejection means (20) for ejecting a predetermined amount of hydrogen peroxide solution onto a surface of the sealing member sealing the accommodating container on a conveyance path of the conveyance means, and an ultrasound emitting means (40) for emitting ultrasounds toward the surface of the sealing member where the hydrogen peroxide solution is ejected, characterized in that
the hydrogen peroxide solution ejected onto the surface of the sealing member by the ejection means allows hydrogen peroxide to vaporize with ultrasounds emitted from the ultrasound emitting means and a vaporizing hydrogen peroxide gas is supplied from the surface of the sealing member to the inside of the accommodating container by receiving ultrasonic sound flows.

Moreover, the present invention is, according to description in claim 2, the decontamination system according to claim 1, characterized in that
the continuous supplying device (100) includes a heating means (30) located at a front stage and/or a rear stage of the ultrasound emitting means on the conveyance path of the conveyance means and heating the hydrogen peroxide solution ejected onto the surface of the sealing member, characterized in that
the hydrogen peroxide solution ejected onto the surface of the sealing member by the ejection means increases in temperature with the heating means, hydrogen peroxide vaporizes with ultrasounds emitted from the ultrasound emitting means, and a vaporizing hydrogen peroxide gas is supplied from the surface of the sealing member to the inside of the accommodating container by receiving ultrasonic sound flows.

Furthermore, the present invention is, according to claim 3, the decontamination system according to claim 1 or 2, characterized in that
the ultrasound emitting means (40) is an ultrasonic vibrating board including a base (41) and a plurality of transmitters (42), characterized in that
the plurality of transmitters is arranged on a plain surface of the base toward the surface of the sealing member so as to be uniform in transmission directions, and the transmitters are operated in the same phase, whereby
a sound flow is generated by a significantly directional ultrasound toward the surface of the sealing member from a board surface of the ultrasonic vibrating board in the vertical direction by mutually amplifying the ultrasounds of the plurality of transmitters in the front direction and mutually canceling out the ultrasounds of the plurality of transmitters in the lateral direction.

Furthermore, the present invention is, according to claim 4, the decontamination system according to claim 1 or 2, characterized in that
the ejection means (20) ejects a hydrogen peroxide solution by dispenser type or ink-jet type, and uniformly applies a small amount of hydrogen peroxide solution over the surface of the sealing member as microscopic liquid droplets.

Furthermore, the present invention is, according to claim 5, the decontamination system according to claim 1 or 2, characterized in that
the ejection means (20) controls the amount of the hydrogen peroxide solution ejected onto the surface of the sealing member such that the amount changes depending on parts on the surface of the sealing member, and
the amount of the vaporizing hydrogen peroxide gas supplied from the surface of the sealing member to the inside of the accommodating container is changed, depending on parts of the pre-filled syringe accommodated inside the accommodating container.

### ADVANTAGEOUS EFFECTS OF INVENTION

According to the above configuration, a decontamination system according to the present invention includes a decontamination agent supply step, a decontamination step and an aeration step. The present invention is characterized by a continuous supplying device used in the decontamination agent supply step, among other things. The continuous supplying device includes a conveyance means, an ejection means and an ultrasound emitting means. The conveyance means conveys an accommodating container from an inlet to an outlet. The ejection means ejects a predetermined amount of hydrogen peroxide solution onto a surface of a sealing member sealing the accommodating container on a conveyance path. The ultrasound emitting means emits ultrasounds on the heated hydrogen peroxide solution toward a surface of the sealing member on the conveyance path of the conveyance means.

Herein, the hydrogen peroxide solution ejected onto the surface of the sealing member by the ejection means allows hydrogen peroxide to vaporize with ultrasounds emitted by the ultrasound emitting means. A vaporizing hydrogen peroxide gas is supplied from the surface of the sealing member to the inside of the accommodating container by receiving ultrasonic sound flows. In this way, according to the above configuration, the present invention can provide a decontamination system capable of continuously decontaminating numerous accommodating containers in small equipment because the consumption of a hydrogen peroxide solution can be reduced within a required range, leading to a smaller amount of the hydrogen peroxide residual inside each accommodating container and significantly shortened decontamination and aeration durations.

According to the above configuration, the continuous supplying device has a heating means. The heating means is located at a front stage and/or a rear stage of the ultrasound emitting means on the conveyance path of the conveyance means and heating the hydrogen peroxide solution ejected onto the surface of the sealing member. Therefore, the hydrogen peroxide solution ejected onto the surface of the sealing member by the ejection means increases in temperature and hydrogen peroxide vaporizes with ultrasounds emitted from the ultrasound emitting means, and a vaporizing hydrogen peroxide gas is supplied from the surface of the sealing member to the inside of the accommodating container by receiving ultrasonic sound flows. Accordingly, the above operational advantage can more specifically be provided.

According to the above configuration, the ultrasound emitting means is an ultrasonic vibrating board including a base and a plurality of transmitters. In the ultrasonic vibrating board, the plurality of transmitters is arranged on a plain surface of the base toward the surface of the sealing member so as to be uniform in transmission directions. By operating these transmitters in the same phase, the ultrasounds of the plurality of transmitters in the front direction are mutually amplified, and the ultrasounds of the plurality of transmitters in the lateral direction are mutually canceled out. Accordingly, a sound flow is generated by a significantly directional ultrasound toward the surface of the sealing member from a board surface of the ultrasonic vibrating board in the vertical direction. Accordingly, the above operational advantage can more specifically be provided.

According to the above configuration, the ejection means ejects a hydrogen peroxide solution by dispenser type or ink-jet type, and uniformly applies a small amount of hydrogen peroxide solution over the surface of the sealing member as microscopic liquid droplets. Accordingly, the above operational advantage can more specifically be provided.

According to the above configuration, the ejection means controls the amount of the hydrogen peroxide solution ejected onto the surface of the sealing member such that the amount changes depending on parts on the surface of the sealing member. Thus, the amount of the vaporizing hydrogen peroxide gas supplied from the surface of the sealing member to the inside of the accommodating container is changed, depending on parts of the pre-filled syringe accommodated inside the accommodating container. Accordingly, the above operational advantage can more specifically be provided.

### BRIEF DESCRIPTION OF DRAWINGS

FIG. 1 is a plan view illustrating a container body of an accommodating container for accommodating a pre-filled syringe to be decontaminated in a decontamination system according to the present invention before it is sealed with a sealing member;
FIG. 2 is a right side view illustrating the container body of the accommodating container for accommodating the pre-filled syringe of FIG. 1 sealed with the sealing member;
FIG. 3 is a front view illustrating the container body of the accommodating container for accommodating the pre-filled syringe of FIG. 1 sealed with the sealing member;
FIG. 4 is a schematic perspective view illustrating the appearance of a continuous supplying device used in the decontamination system according to the present invention;
FIG. 5 is a side cross-sectional view illustrating the inside of the continuous supplying device of FIG. 4; and
FIG. 6(A) is a front view illustrating the accommodating container in the decontamination system using the continuous supplying device of FIG. 4 in a decontamination agent supply step prior to each operation, FIG. 6(B) illustrates the same during an ejection operation, FIG. 6(C) illustrates the same during a heating operation, FIG. 6(D) illustrates the same during an ultrasonic operation, and after the decontamination agent supply step, FIG. 6(E) illustrates the same in a decontamination step, and FIG. 6(F) illustrates the same in an aeration step.

### DESCRIPTION OF EMBODIMENTS

A decontamination system according to the present invention will be described with reference to an embodiment. The present invention is not restricted to the following embodiment. In the decontamination system according to the following embodiment, a decontamination agent used is a hydrogen peroxide solution. First, an article to be decontaminated by a hydrogen peroxide solution will be described. In this embodiment, external surfaces of a pre-filled syringe accommodated in an accommodating container and the inside of the accommodating container are to be decontaminated.

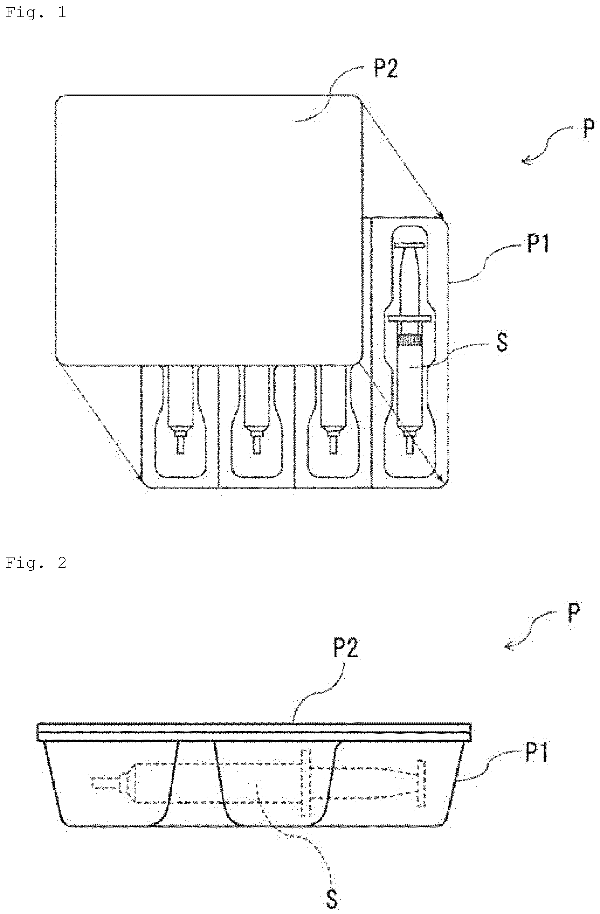

FIG. 1 is a plan view illustrating a container body of an accommodating container for accommodating a pre-filled syringe to be decontaminated in a decontamination system according to the present invention before it is sealed with a sealing member. In FIG. 1, an accommodating container P includes a polyethylene container body P1 and a sealing member P2 of Tyvek (trademark). A pre-filled syringe S is accommodated within the container body P1. In FIG. 1, the accommodating container P containing a set of 4 pre-filled syringes S is used, but the configuration is not restricted to this. Also, FIG. 1 illustrates the state of the container body P1 and the sealing member P2 before being sealed.

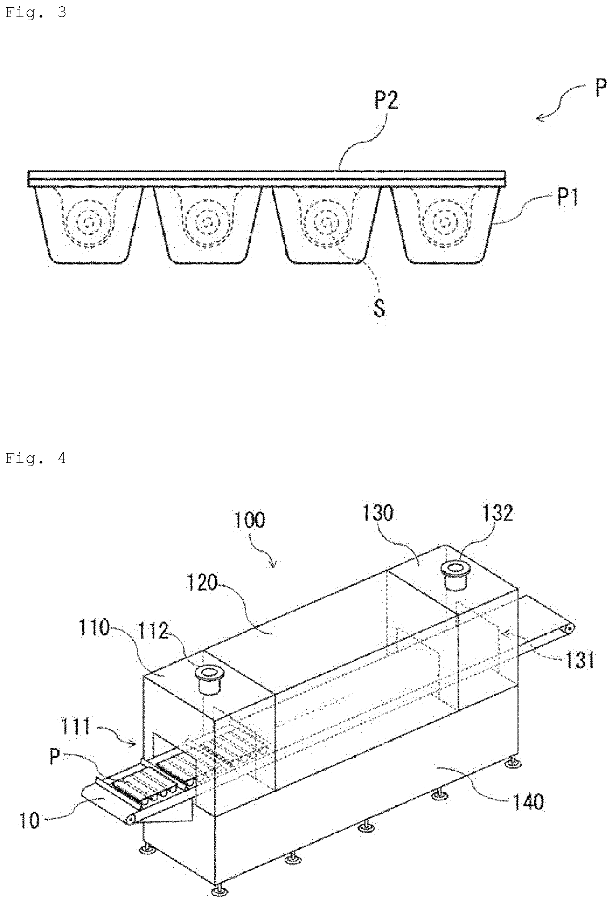

FIG. 2 is a right side view illustrating the container body of the accommodating container for accommodating the pre-filled syringe of FIG. 1 sealed with the sealing member. FIG. 3 is a front view illustrating the container body of the accommodating container for accommodating the pre-filled syringe of FIG. 1 sealed with the sealing member. In FIGS, 2 and 3, the inside of the accommodating container P and the pre-filled syringe S accommodated therein are not decontaminated.

The decontamination system according to this embodiment is composed of a decontamination agent supply step, a decontamination step and an aeration step. The decontamination system according to this embodiment is characterized by a decontamination agent supply step. The decontamination agent supply step will mainly be described.

In this embodiment, a decontamination process is performed by generating hydrogen peroxide gas from a 35 W/V% hydrogen peroxide solution used as a decontamination agent. Advantageously, the hydrogen peroxide gas has a strong sterilization effect, and is inexpensively available and effectively utilized as an environmentally-friendly decontamination gas that is ultimately resolved into oxygen and water.

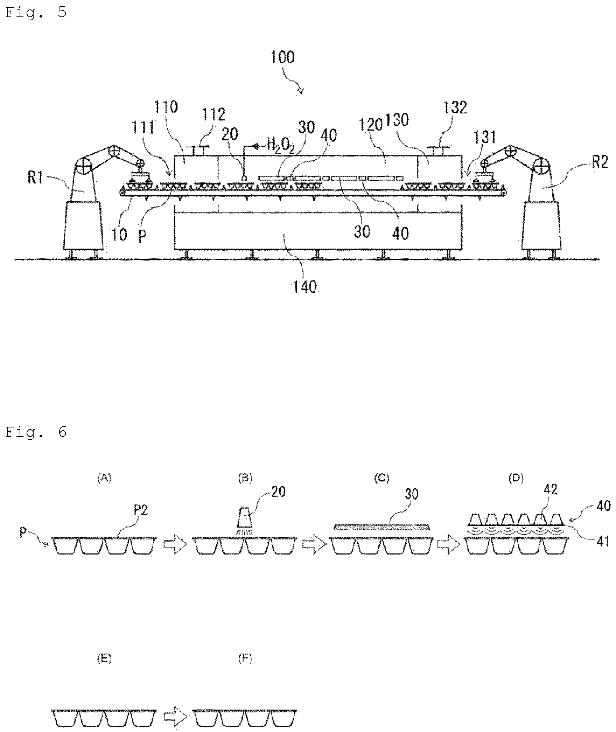

Next, a continuous supplying device used in the decontamination agent supply step will be described. FIG. 4 is a schematic perspective view illustrating the appearance of the continuous supplying device. FIG. 5 is a side cross-sectional view illustrating the inside of the continuous supplying device. A continuous supplying device 100 according to this embodiment converts a hydrogen peroxide solution as decontamination agent into gas and supplies the resulting gas to the inside of the accommodating container P accommodating the pre-filled syringe S to be decontaminated.

In FIGS. 4 and 5, the continuous supplying device 100 is composed of a box body configured by a wall portion formed of a stainless metal plate and placed on the floor. The inside of the continuous supplying device 100 is divided into a front chamber 110, a decontamination agent supply chamber 120 and a rear chamber 130 by each wall portion. Also, electric equipment, machines and so on are accommodated inside a mount portion 140 of the continuous supplying device 100.

The continuous supplying device 100 includes a conveyance conveyor 10 running from the outside of an inlet 111, through the front chamber 110, the decontamination agent supply chamber 120 and the rear chamber 130 to the outside of an outlet 131 to convey the accommodating container P. In this embodiment, the conveyance means 10 used is a conveyor. On a conveyance path of the conveyance conveyor 10, an ejection device 20, a heater 30 and an ultrasonic vibrating board 40 are provided in the decontamination agent supply chamber 120. In this embodiment, the heater 30 is provided, but the configuration is not restricted to this, and only the ultrasonic vibrating board 40 may be available (as later described).

The operation of each apparatus with respect to the accommodating container P conveyed by the conveyance conveyor 10 will be described. In FIG. 5, the inside of the front chamber 110 and the rear chamber 130 is maintained in a negative pressure because an air exhaust device (not shown) removes air from air outlets 112, 132, respectively. This operation is to prevent part of the hydrogen peroxide solution supplied by the decontamination agent supply chamber 120 from being discharged toward the external environment. In this state, the conveyance conveyor 10 is rotated at a constant speed rightward in the figure.

A robot arm of a carry-in device R1 external to the inlet 111 places the accommodating container P, with the sealing member P2 facing upward, on a belt of the conveyance conveyor 10. The accommodating container P of the conveyance conveyor 10 passes from the inlet 111, through the front chamber 110 and to the decontamination agent supply chamber 120. In the decontamination agent supply chamber 120, while the accommodating container P moves under the ejection device 20 by the conveyance conveyor 10, a hydrogen peroxide solution is ejected from the ejection 20 onto a surface of the sealing member P2.

Herein, the ejection device 20 will be described. In the present invention, the type of the ejection 20 is not particularly restricted. Thus, spray nozzles or two-fluid nozzles can be employed. In order to uniformly apply a particularly small amount of hydrogen peroxide solution over the surface of the sealing member P2, dispenser type or ink-jet type ejection is preferable.

In this case, the ejection type is not particularly restricted, and any of air pulse type, plunger type, screw pump type, piezo type, thermal type and bubble type may be available. In addition, the discharge apparatus may be fixed using a plurality of nozzles corresponding to the width of the sealing member P2, or may be movable by traversing along the width of the sealing member P2. In this embodiment, an ejection nozzle of ink-jet type is employed.

In this embodiment, the amount of pre-discharged hydrogen peroxide solution ejected and applied is preferably determined in the range of, for example, 0.05 mg/cm2 to 2 mg/cm2 based on the internal volume of the accommodating container P where the pre-filled syringe S is accommodated and the surface area of its corresponding sealing member P2. In this embodiment, the ejection nozzle of ink-jet type can be employed to uniformly apply a predetermined amount of hydrogen peroxide solution over the surface of the sealing member P2. A larger amount of hydrogen peroxide solution may be applied, but the following aeration operation unfortunately needs a longer duration, resulting in a prolonged entire operation.

In this embodiment, a hydrogen peroxide solution is uniformly applied over the surface of the sealing member P2, but the configuration is not restricted to this, and the amount of hydrogen peroxide solution discharged toward the surface of the sealing member P2 may be controlled depending on parts on the surface of the sealing member P2 (as later described).

In this way, while the accommodating container P where the hydrogen peroxide solution is uniformly applied over the surface of the sealing member P2 moves under the heater 30 by the conveyance conveyor 10, the hydrogen peroxide solution applied over the surface of the sealing member P2 is heated by the heater 30.

Herein, the heater 30 will be described. The heater 30 is disposed to be parallel to the surface of the sealing member P2, and the hydrogen peroxide solution applied over the surface of the sealing member P2 is uniformly heated to increase the temperature. In the present invention, the type of the heater 30 is not particularly restricted. In this embodiment, a far-infrared heater or a infrared laser is preferably used due to short-time and uniform heating. In addition, the surface temperature of the sealing member P2, corresponding to the heated temperature of the hydrogen peroxide solution, is preferably determined in the range of, for example, 30 °C to 50 °C. The temperature may further be increased, but such temperature increase can adversely affect a chemical filled in the pre-filled syringe S.

In this way, while the accommodating container P, with the hydrogen peroxide solution applied over the surface of the sealing member P2 uniformly heated, moves under the ultrasonic vibrating board 40 by the conveyance conveyor 10, ultrasounds are emitted on the hydrogen peroxide solution heated on the surface of the sealing member P2. The hydrogen peroxide solution applied over the surface of the sealing member P2 is converted into gas from the surface of the sealing member P2 by receiving ultrasonic sound flows and supplied to the inside of the accommodating container P.

In this embodiment, a plurality of heaters 30 and a plurality of ultrasonic vibrating boards 40 are alternately disposed rearward of the ejection device 20 of the decontamination agent supply chamber 120. Accordingly, the hydrogen peroxide solution applied over the surface of the sealing member P2 is repeatedly subjected to heating and ultrasonic irradiation, the applied hydrogen peroxide solution completely vaporizes, and is supplied from the surface of the sealing member P2 to the inside of the accommodating container P by receiving ultrasonic sound flows. In this embodiment, the plurality of heaters 30 and the plurality of ultrasonic vibrating boards 40 are alternately disposed, but the configuration is not restricted to this (as later described).

In this way, the hydrogen peroxide solution applied over the surface of the sealing member P2 is converted into hydrogen peroxide gas and supplied to the inside of the accommodating container P by receiving ultrasonic sound flows to allow the accommodating container P to pass through the rear chamber 130 and out of the outlet 131 by the conveyance conveyor 10. A robot arm of a carry-in device R2 external to the outlet 131 carries the accommodating container P out of the conveyance conveyor 10.

Herein, the operational advantage of the ejection device 20, the heater 30 and the ultrasonic vibrating board 40 of the continuous supply device 100 will be described. FIG. 6(A) is a front view illustrating the accommodating container in the decontamination system using the continuous supplying device in a decontamination agent supply step prior to each operation, FIG. 6(B) illustrates the same during an ejection operation, FIG. 6(C) illustrates the same during a heating operation, FIG. 6(D) illustrates the same during an ultrasonic operation, and after the decontamination agent supply step, FIG. 6(E) illustrates the same in a decontamination step, and FIG. 6(F) illustrates the same in an aeration step.

### <<Decontamination agent supply step>>

First, FIG. 6 (A) illustrates the state before the accommodating container P is carried into the decontamination agent supply chamber 120. In this state, no hydrogen peroxide solution is applied over the surface of the sealing member P2. Next, FIG. 6 (B) illustrates the state where a hydrogen peroxide solution is ejected onto the surface of the sealing member P2 from the ejection device 20. As described above, by using an ink-jet type ejection nozzle, a predetermined amount of hydrogen peroxide solution can uniformly be applied over the surface of the sealing member P2.

Next, FIG. 6 (C) illustrates the state where the hydrogen peroxide solution applied over the surface of the sealing member P2 is heated by the heater 30 and ejected. As described above, by using a far-infrared heater and so on, the surface temperature of the sealing member P2 can be increased in a range of 30 °C to 50 °C. Next, FIG. 6 (D) illustrates the state of ultrasonic emitting from the ultrasonic vibrating board 40 toward the surface of the sealing member P2.

Herein, the ultrasonic vibrating board 40 will be described. The ultrasonic vibrating board 40 is disposed to be parallel to the surface of the sealing member P2 to irradiate with ultrasound the hydrogen peroxide solution applied over the surface of the sealing member P2 and heated. In this embodiment, the ultrasonic vibrating board 40 includes a base (speaker base) 41 and a plurality of transmitters (ultrasonic speaker) 42 provided on a plain surface of the base. Also, the plurality of transmitters (ultrasonic speaker) 42 is arranged to face the surface of the sealing member P2 so as to be uniform in transmission direction.

In this state, the plurality of transmitters (ultrasonic speaker) 42 is operated in the same phase. Accordingly, the ultrasounds of the plurality of transmitters (ultrasonic speaker) 42 in the front direction are mutually amplified, and the ultrasounds of the plurality of transmitters (ultrasonic speaker) 42 in the lateral direction are mutually canceled out. Consequently, a sound flow can be generated by a significantly directional ultrasound from a board surface of the ultrasonic vibrating board 40 toward the surface of the sealing member P2 in the vertical direction.

In this way, when the surface of the sealing member P2 is irradiated with sound flows by the significantly directional ultrasound from the ultrasonic vibrating board 40, the hydrogen peroxide solution applied over the surface of the sealing member P2 and heated vaporizes by receiving ultrasonic energy, and is converted into hydrogen peroxide gas. Further, the vaporizing hydrogen peroxide gas is supplied from the surface of the sealing member P2 to the inside of the accommodating container P by receiving ultrasonic sound flows.

### <<Decontamination step>>

Next, FIG. 6(E) shows the state of a decontamination step.
Through repetitive heating and ultrasonic operations after the ejection operation inside the decontamination agent supply chamber 120, a predetermined amount of hydrogen peroxide gas is supplied to the inside of the accommodating container P. Despite the start of decontamination inside the accommodating container P in the middle of the decontamination agent supply step, the accommodating container P carried out of the conveyance conveyor 10 is stored for a predetermined duration to complete the decontamination step.

### <<Aeration step>>

Next, FIG. 6 (F) illustrates the state of an aeration step. In this embodiment, the supply of a small and proper amount of hydrogen peroxide solution can result in no hydrogen peroxide being generally residual inside the accommodating container P at the end of the decontamination step. However, by replacing the air inside the chamber accommodating the accommodating container P after the decontamination step with clean air, more perfect aeration step is finalized. In addition, by heating the inside of the chamber during the aeration process, the duration of the aeration step can be shortened.

As described above, according to the above embodiment, the present invention can provide a decontamination system capable of continuously decontaminating numerous accommodating containers in small equipment because the consumption of a hydrogen peroxide solution can be reduced within a required range, leading to a smaller amount of the hydrogen peroxide residual inside each accommodating container and significantly shortened decontamination and aeration durations.

The present invention is achieved not only by the above embodiment, but also by the following various alternatives.
(1) In the above embodiment, the heater 30 is placed, but the configuration is not restricted to this. In the present invention, since the amount of hydrogen peroxide solution applied over a surface of a sealing member P2 is proper and small, the hydrogen peroxide solution can vaporize not by being heated by a heater 30, but only by the action of an ultrasonic vibrating board 40.
(2) In the above embodiment, a plurality of heaters 30 and a plurality of ultrasonic vibrating boards 40 are alternately disposed, but the configuration is not restricted to this. In this case, instead of a plurality of heaters 30 and a plurality of ultrasonic vibrating boards 40 being placed, heaters 30 may be placed only at the front stage of ultrasonic vibrating boards 40 or only at the rear stage of the ultrasonic vibrating boards 40.
(3) In the above embodiment, a hydrogen peroxide solution is uniformly applied over a surface of a sealing member P2, but the configuration is not restricted to this. For example, the amount of a hydrogen peroxide solution ejected onto the surface of the sealing member P2 may be controlled such that the amount changes depending on parts on the surface of the sealing member P2. A pre-filled syringe S accommodated inside the accommodating container P has a varied amount of hydrogen peroxide gas required for decontamination, depending on the structure of each part thereof. Thus, the amount of hydrogen peroxide solution required for decontamination is preferably controlled to be proper and small, depending on the structure of each part of the pre-filled syringe S.

### REFERENCE SIGNS LIST

P...Accommodating container, P1...Container body, P2...Sealing member, R1...Carry-in device,
R2...Discharge device, S...Pre-filled syringe, 10...Conveyance conveyor,
20...Discharge device, 30...Heater, 40...Ultrasonic vibrating board,
41...Base (Speaker base), 42...Transmitter (Ultrasonic speaker),
100...Continuous supplying device, 110...Front chamber,
120...Decontamination agent supply chamber, 130...Rear chamber,
111...Inlet, 131...Outlet, 112, 132...Air outlet, 140...Mount portion.

## Claims

1. A decontamination system for decontaminating external surfaces of a pre-filled syringe accommodated inside an accommodating container, composed of a container body and a gas-permeable sealing member for sealing a released portion of the container body, with the pre-filled syringe being accommodated, the system comprising:
a decontamination agent supply step of supplying a decontamination agent to the inside of the accommodating container using a continuous supplying device; a decontamination step of maintaining the accommodating container supplied with the decontamination agent for a predetermined duration and decontaminating external surfaces of the pre-filled syringe; and an aeration step of removing the residual decontamination agent from the inside of the accommodating container after decontamination and replacing the agent with clean air, wherein
the continuous supplying device includes a conveyance means for conveying the accommodating container from an inlet to an outlet, an ejection means for ejecting a predetermined amount of hydrogen peroxide solution onto a surface of the sealing member sealing the accommodating container on a conveyance path of the conveyance means, and an ultrasound emitting means for emitting ultrasounds toward the surface of the sealing member where the hydrogen peroxide solution is ejected, wherein
the hydrogen peroxide solution ejected onto the surface of the sealing member by the ejection means allows hydrogen peroxide to vaporize with ultrasounds emitted from the ultrasound emitting means and a vaporizing hydrogen peroxide gas is supplied from the surface of the sealing member to the inside of the accommodating container by receiving ultrasonic sound flows.

2. The decontamination system according to claim 1, wherein
the continuous supplying device has a heating means located at a front stage and/or a rear stage of the ultrasound emitting means on the conveyance path of the conveyance means and heating the hydrogen peroxide solution ejected onto the surface of the sealing member, wherein the hydrogen peroxide solution ejected onto the surface of the sealing member by the ejection means increases in temperature with the heating means, hydrogen peroxide vaporizes with ultrasounds emitted from the ultrasound emitting means, and a vaporizing hydrogen peroxide gas is supplied from the surface of the sealing member to the inside of the accommodating container by receiving ultrasonic sound flows.

3. The decontamination system according to claim 1 or 2, wherein
the ultrasound emitting means is an ultrasonic vibrating board including a base and a plurality of transmitters, wherein
the plurality of transmitters is arranged on a plain surface of the base toward the surface of the sealing member so as to be uniform in transmission directions, and the transmitters are operated in the same phase, whereby
a sound flow is generated by a significantly directional ultrasound toward the surface of the sealing member from a board surface of the ultrasonic vibrating board in the vertical direction by mutually amplifying the ultrasounds of the plurality of transmitters in the front direction and mutually canceling out the ultrasounds of the plurality of transmitters in the lateral direction.

4. The decontamination system according to claim 1 or 2, wherein
the ejection means ejects a hydrogen peroxide solution by dispenser type or ink-jet type, and uniformly applies a small amount of hydrogen peroxide solution over the surface of the sealing member as microscopic liquid droplets.

5. The decontamination system according to claim 1 or 2, wherein
the ejection means controls the amount of the hydrogen peroxide solution ejected onto the surface of the sealing member such that the amount changes depending on parts of the surface of the sealing member, and
the amount of the vaporizing hydrogen peroxide gas supplied from the surface of the sealing member to the inside of the accommodating container is changed, depending on parts of the pre-filled syringe accommodated inside the accommodating container.
